(19) **Europäisches Patentamt**

**European Patent Office**

**Office européen des brevets**

(11) **EP 0 858 987 B1**

(12) **FASCICULE DE BREVET EUROPEEN**

(45) Date de publication et mention
de la délivrance du brevet:
**04.10.2000 Bulletin 2000/40**

(51) Int Cl.$^7$: **C07C 2/00**

(21) Numéro de dépôt: **98400208.9**

(22) Date de dépôt: **02.02.1998**

(54) **Procédé de conversion d'alcanes légers en hydrocarbures supérieurs**

Verfahren zur Umsetzung von leichten Kohlenwasserstoffen in höheren Kohlenwasserstoffen

Process for the conversion of lighter alkanes to higher hydrocarbons

(84) Etats contractants désignés:
**AT BE CH DE DK ES FI FR GB GR IE IT LI LU NL PT SE**

(30) Priorité: **18.02.1997 FR 9701866**

(43) Date de publication de la demande:
**19.08.1998 Bulletin 1998/34**

(73) Titulaire: **TOTAL RAFFINAGE DISTRIBUTION S.A.**
**92800 Puteaux (FR)**

(72) Inventeurs:
• **Amariglio, Henri**
**54000 Nancy (FR)**
• **Pareja, Pierre**
**54600 Villers-Les-Nancy (FR)**
• **Szabo, Georges**
**76290 Montivilliers (FR)**

(74) Mandataire: **Jolly, Jean-Pierre et al**
**Cabinet Jolly**
**54, rue de Clichy**
**75009 Paris (FR)**

(56) Documents cités:
**WO-A-92/01656**

• **PAREJA P ET AL: "INCREASING THE YIELD IN METHANE HOMOLOGATION THROUGH AN ISOTHERMAL TWO-REACTION SEQUENCE AT 250<SYMBOL**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

**Description**

La présente invention concerne un procédé de conversion d'hydrocarbures légers gazeux, comme le gaz naturel par exemple, en hydrocarbures supérieurs comprenant majoritairement des composés cycliques.

L'abondance des champs de gaz naturel a amené les sociétés pétrolières à étudier la possibilité de convertir ce gaz sur le champ même, en des hydrocarbures supérieurs liquéfiables à température ambiante, par des réactions de déshydrogénation en présence d'un catalyseur. Parmi ces réactions, les plus simples sont les réactions visant la conversion du méthane en paraffines supérieures :

$$nCH_4 \rightarrow C_nH_{(2n+2)} + (n-1)H_2$$

Cependant, ce type de réaction très endothermique ne peut s'effectuer de façon notable qu'à des températures élevées, supérieures à 500°C, comme le montre le tableau suivant :

| Température (°C) | Conversion maximum du méthane à l'équilibre thermodynamique (%) |
|---|---|
| 27 | 0,0002 |
| 127 | 0,0067 |
| 227 | 0,0642 |
| 327 | 0,35 |
| 427 | 1,31 |
| 527 | 3,85 |
| 627 | 9,35 |
| 727 | 19,1 |

Ainsi la demande de brevet EP-A-0 192 289 préconise la conversion directe du gaz naturel en hydrocarbures aromatiques, à température élevée, sur un catalyseur à base de silicate alcalin et contenant de l'aluminium et/ou du gallium. Toutefois, cette conversion du méthane s'avère peu sélective et entraîne la formation de coke.

Dans le but de pallier ces inconvénients, une tentative a été envisagée dans un article de la revue "Catalysis Today" 21, 1994, p.423-430, intitulé "Increasing the yield in methane homologation through an isothermal two-reaction sequence at 250°C on platinum", qui décrit un procédé en deux étapes :

- dans la première étape, on met le catalyseur en contact avec le méthane à la pression atmosphérique et à une température de l'ordre de 250°C. Le méthane se chimisorbe de façon dissociative sur les sites métalliques du catalyseur supporté, en fragments ($CH_x$) et en hydrogène (yH), où x+y = 4, avec une déshydrogénation progressive qui dépend notamment de la température, du métal du catalyseur, du débit de méthane ; aux températures préconisées, de l'ordre de 250°C, l'hydrogène se désorbe et est entraîné par le méthane.
- dans la seconde étape, le catalyseur ainsi traité est mis en contact avec de l'hydrogène, toujours à la pression atmosphérique, et les hydrocarbures supérieurs formés par hydrogénation sont désorbés et récupérés.

De plus, la demande de brevet WO-A-92/01656 préconise, pour ce type de conversion, l'utilisation d'un métal de transition sur un support constitué essentiellement d'un oxyde métallique réfractaire, à une température comprise entre 100 et 300°C : on fait passer alternativement sur le catalyseur un flux de méthane, puis un flux d'hydrogène ou, inversement, on fait circuler le catalyseur successivement dans le méthane, puis dans l'hydrogène, le catalyseur se présentant alors sous la forme d'un lit fluidisé circulant. Lorsque la réaction de conversion est achevée, on recueille un flux gazeux composé d'hydrogène et d'un mélange d'alcanes légers ($C_2$ à $C_4$), qui constituent les produits recherchés. Le pourcentage d'alcanes légers dans les hydrocarbures récupérés ne dépasse pas 20 %, le reste étant recueilli sous forme de méthane, ce qui ne présente que peu d'intérêt.

Contrairement aux types de produits que permettent d'obtenir ces techniques antérieures, la Demanderesse a trouvé qu'il était plus intéressant de chercher à obtenir non pas des hydrocarbures légers et gazeux aux conditions normales de température et de pression, mais des hydrocarbures plus lourds, et liquides dans les même conditions.

Dans ce but, la Demanderesse s'est intéressée à un procédé en deux étapes comme celui décrit plus haut et elle a constaté, de manière surprenante, que le fait de réaliser l'étape d'adsorption à température suffisante tout en opérant à pression élevée dans l'une au moins des deux étapes permet d'obtenir in fine une quantité améliorée d'hydrocarbures comportant 6 atomes de carbone ou plus (dénommés $C_{6+}$ dans la suite de la description), et plus principalement des hydrocarbures cycliques, saturés ou non saturés.

En particulier, la Demanderesse a constaté que le fait de réaliser l'étape de désorption à pression élevée permet d'accroître, de manière très significative, l'efficacité du procédé. Ceci permet en effet d'obtenir une quantité accrue d'hydrocarbures ayant au moins 5 atomes de carbone ($C_{5+}$).

L'invention propose donc un choix de conditions opérationnelles nouvelles pour la mise en oeuvre d'un procédé utilisant un catalyseur supporté que l'on place alternativement en présence d'un mélange d'hydrocarbures gazeux naturels pour effectuer la première étape d'adsorption par chimisorption/déshydrogénation des hydrocarbures, puis en présence d'un gaz approprié pour effectuer la deuxième étape de désorption et de récupération des hydrocarbures supérieurs formés.

En outre, la Demanderesse préconise l'utilisation préférentielle de différents types de catalyseurs de façon à augmenter davantage la quantité d'hydrocarbures $C_{5+}$ récupérés, soit en sélectionnant la nature du métal ou de l'alliage métallique supporté, soit en sélectionnant la nature du support.

Un des avantages du procédé selon l'invention est en particulier d'éviter une étape d'adsorption trop longue qui pourrait entraîner une déshydrogénation excessive des hydrocarbures légers adsorbés sur le catalyseur ainsi que le dépôt de carbone irréversible et indésirable pouvant en résulter.

L'invention a par conséquent pour objet un procédé conversion d'un mélange d'hydrocarbures légers, gazeux aux conditions normales de température et de pression, contenant du méthane, en hydrocarbures supérieurs, liquides aux conditions normales de température et de pression, ce procédé comportant deux étapes, une étape d'adsorption dudit mélange sur un catalyseur supporté métallique, suivie d'une étape de désorption des espèces adsorbées. Ce procédé se caractérise en ce que l'étape d'adsorption est réalisée à température supérieure ou égale à 300 °C et en ce que au moins l'une des deux étapes est réalisée sous une pression supérieure ou égale à $5.10^5$ Pa.

L'étape d'adsorption selon la présente invention est une véritable réaction chimique entre la surface métallique du catalyseur et les hydrocarbures légers, accompagnée d'un dégagement d'hydrogène : les espèces hydrocarbures déshydrogénées produites lors de l'adsorption, que l'on peut noter ($CH_X$), sont stockées sur la surface du catalyseur et réagissent entre elles de façon à former les précurseurs des molécules que l'on désire obtenir.

Lors de l'étape de désorption, ces espèces hydrocarbures stockées sont recueillies sous forme d'hydrocarbures supérieurs, liquides aux conditions normales de température et de pression, principalement de type $C_{6+}$, cycliques, saturés ou non saturés. La désorption peut avantageusement consister en une hydrogénation plus ou moins poussée des espèces stockées sur la surface du catalyseur, par introduction d'hydrogène.

Comme catalyseur, on pourra utiliser tout catalyseur possédant une fonction hydrogénante/déshydrogénante manifestant une grande sélectivité de cette fonction vis-à-vis de l'hydrogénolyse indésirable, par exemple les catalyseurs contenant un ou plusieurs métaux (alliage) déposé(s) sur un support, de préférence un oxyde de gallium, de titane, de thorium, de bore, un mélange de ces oxydes, une silice, une alumine, une silice-alumine, une silicalite, un alumino-silicate, une magnésie ou une zircone.

Les métaux ou alliages choisis pour ces catalyseurs pourront être les métaux du groupe VIII de la Classification Périodique des éléments et en particulier ceux choisis parmi le platine, le cobalt, le nickel, le rhodium, le cuivre, ou un alliage de ces métaux.

Les alliages nickel/cuivre présentent une bonne activité et une sélectivité plus élevée que le nickel pur, qui peut manifester une hydrogénolyse parfois excessive.

Le nombre de sites actifs accessibles sur le support par unité de surface et par conséquent la teneur en métal déposé sur ce support doivent être élevés, de manière à pouvoir adsorber le maximum d'espèces hydrocarbures pendant l'étape d'adsorption. On retiendra pour le catalyseur des teneurs en métal comprises entre 5 et 50 % en poids par rapport au poids du support.

Les caractéristiques du catalyseur conditionnent également l'accessibilité des sites actifs aux molécules d'hydrocarbures à convertir. On retiendra donc une surface spécifique élevée, supérieure ou égale à 200 m²/g, et/ou un volume poreux élevé, supérieur ou égal à 0,2 cm³/g.

Les catalyseurs utilisés dans la présente invention sont préparés selon les méthodes classiques connues, en déposant les précurseurs des métaux sur le support, puis en calcinant les solides ainsi obtenus. Puis une étape de réduction, utilisant de l'hydrogène, par exemple, sera pratiquée dans des conditions appropriées avant la mise en oeuvre du catalyseur.

Les conditions opérationnelles du procédé selon l'invention seront choisies de façon à obtenir le maximum de produits liquides aux conditions normales de température et de pression.

L'étape réalisée sous pression supérieure ou égale à $5.10^5$ Pa peut être l'étape d'adsorption seule, ou de préférence l'étape de désorption seule. En effet, une pression élevée lors de la désorption permet une récupération accrue de la matière adsorbée sur le catalyseur.

De manière encore plus avantageuse, les deux étapes sont réalisées sous pression supérieure ou égale à $5.10^5$ Pa. L'étape de désorption peut alors s'effectuer à la même pression que l'étape d'adsorption des hydrocarbures légers à convertir, ou à une pression différente et de préférence plus élevée.

De préférence, les étapes effectuées sous pression supérieure ou égale à $5.10^5$ Pa sont réalisées sous une pression comprise entre $10.10^5$ et $80.10^5$ Pa.

La température lors de l'étape d'adsorption est de préférence comprise entre 350 et 500 °C.

L'étape d'adsorption s'effectue pendant une durée comprise entre 3 secondes et 2 minutes, de préférence entre 3 secondes et 1 minute.

De manière avantageuse, on pourra conduire l'adsorption en présence d'une faible quantité d'hydrogène dans le mélange d'hydrocarbures légers, pour bien maîtriser la déshydrogénation des espèces qui s'adsorbent, sans aller jusqu'à la formation d'espèces trop riches en carbone et non régénérables lors de l'étape ultérieure de désorption.

L'étape de désorption peut s'effectuer à la même température que l'étape d'adsorption des hydrocarbures à convertir. Elle peut aussi et de manière avantageuse s'effectuer à température inférieure à celle de l'étape d'adsorption, mais, dans ce cas, il est souhaitable que la température de l'étape de désorption soit supérieure ou égale à 100 °C. Cette température peut être constante durant l'étape de désorption, et est alors avantageusement comprise entre 100 et 400°C, pour limiter l'hydrogénolyse et conduire à des hydrocarbures liquides.

Cette seconde étape peut également être conduite selon un procédé polytherme c'est-à-dire en faisant croître la température de manière progressive, de préférence dans l'intervalle de température compris entre 100 et 600 °C.

La désorption s'effectue pendant une durée comprise entre 1 seconde et 10 minutes, de préférence entre 5 secondes et 2 minutes.

Avantageusement, la désorption est réalisée par hydrogénation, en introduisant de l'hydrogène pur ou en mélange avec un ou plusieurs hydrocarbure(s) et/ou un ou plusieurs gaz inerte(s).

Des alternatives à la désorption hydrogénante peuvent également être envisagées, comme par exemple la désorption par balayage de monoxyde de carbone ou d'un autre gaz approprié.

De manière avantageuse, les hydrocarbures légers produits et se trouvant à l'état non liquide aux conditions normales de température et de pression, sont recyclés partiellement ou totalement, en mélange avec le mélange d'hydrocarbures légers constituant la charge, ou séparément.

L'hydrogène formé durant l'adsorption peut être partiellement, ou pour l'essentiel, éliminé du milieu réactionnel in situ par un dispositif connu en soi, tel qu'un dispositif membranaire.

Le procédé conforme à l'invention sera de préférence mis en oeuvre de façon continue. On peut procéder au moyen d'un lit fixe de catalyseur, sur lequel circulent alternativement un flux d'hydrocarbures légers à convertir puis un flux de gaz (hydrogène par exemple) permettant de réaliser la désorption des hydrocarbures supérieurs formés.

On peut également faire circuler le catalyseur entre un premier réacteur d'adsorption du méthane et un second réacteur de désorption (par exemple d'hydrogénation), puis retour au premier réacteur et ainsi de suite.

L'hydrogène présent dans le flux gazeux sortant du réacteur lors de l'étape d'adsorption est séparé, pour l'essentiel, des hydrocarbures légers non adsorbés, par un ou plusieurs dispositifs classiques tel que des membranes, par adsorption/désorption alternées sur zéolithes, ou par cryogénie.

Les hydrocarbures légers gazeux non adsorbés lors de cette première étape sont avantageusement recyclés en tant que charge à convertir.

De même, l'issue de l'étape de désorption hydrogénante, les produits formés sont séparés de l'hydrogène. On récupère ensuite les produits liquides et l'on peut recycler, séparément ou en mélange avec le mélange d'hydrocarbures légers constituant la charge, les hydrocarbures gazeux légers également produits.

On utilisera, pour ces différentes séparations, les méthodes de condensation, d'extraction et de piégeage bien connues de l'homme du métier.

Le procédé selon l'invention présente l'avantage de conduire aux hydrocarbures liquides et à l'hydrogène gazeux, les hydrocarbures gazeux légers (éthane, propane, butane) étant recyclés.

Les exemples qui suivent illustrent l'invention sans toutefois la limiter.

Ces exemples visent à montrer que le procédé selon l'invention permet d'obtenir une bonne conversion des hydrocarbures légers, gazeux aux conditions normales de température et de pression, en hydrocarbures lourds, liquides dans les mêmes conditions.

Dans tous ces exemples, la charge que l'on souhaite convertir est constituée de méthane pur et le procédé de conversion s'effectue en deux étapes, l'une d'adsorption du méthane sur le catalyseur, l'autre de désorption hydrogénante des espèces adsorbées.

EXEMPLE 1

Dans cet exemple, on utilise un catalyseur supporté bimétallique nickel/cuivre sur silice. Ce catalyseur contient 16 % en poids de métaux et son rapport atomique, Cu/(Cu + Ni), est égal à 0,5. Il présente une surface spécifique de 230 $m^2$/g et un volume poreux de 0,6 $cm^3$/g.

La récupération des produits se fait ici par hydrogénation polytherme, c'est-à-dire par hydrogénation en montée progressive de la température, depuis la température d'adsorption jusqu'à 600 °C, sous un débit d'hydrogène de 200 ml/min et sous une pression de $32.10^5$ Pa, en piégeant les hydrocarbures au fur et à mesure de leur formation.

Le débit gazeux est un débit volumique mesuré aux conditions normales de température et de pression.

Le Tableau I ci-après rassemble, pour différentes conditions opératoires et en utilisant à chaque fois 100 mg de catalyseur, les résultats de la conversion du méthane, en donnant la sélectivité en hydrocarbures $C_{5+}$ parmi les hydrocarbures supérieurs recueillis (rapport, exprimé en %, de $C_{5+}$ sur $C_{2+}$).

## Tableau I

| Conditions d'adsorption | | | | | Sélectivité |
|---|---|---|---|---|---|
| Température (°C) | Pression (Pa) | Débit (ml/min) | Durée (secondes) | $CH_4$ envoyé sur le catalyseur (mg) | ($C_{5+}/C_{2+}$ en %) |
| 350 | $16.10^5$ | 1600 | 18 | 343 | 81 |
| 350 | $16.10^5$ | 1600 | 12 | 229 | 82 |
| 350 | $16.10^5$ | 1600 | 36 | 686 | 75 |
| 400 | $16.10^5$ | 1600 | 6 | 114 | 66 |

Ce tableau illustre les excellents résultats apportés par un choix judicieux des conditions de température et de pression, tel que proposé dans l'invention. Ainsi, en réalisant l'étape d'adsorption à des pression et température élevées puis l'étape de désorption à pression élevée ($32.10^5$ Pa) et à température croissante au cours du temps, on obtient une très bonne sélectivité de la conversion du méthane en faveur des hydrocarbures lourds liquides de type $C_{5+}$.

EXEMPLE 2

Cet exemple est similaire à l'exemple 1, mais il est réalisé en effectuant l'étape de désorption par une hydrogénation à température constante et identique à celle de l'étape d'adsorption, avec un débit d'hydrogène pur de 200 ml/min, et une pression de $4.10^5$ Pa ou $16.10^5$ Pa selon les essais. Le débit gazeux est un débit volumique mesuré aux conditions normales de température et de pression.

Le Tableau II ci-après rassemble pour différentes conditions opératoires et en utilisant 100 mg de catalyseur les résultats de la conversion du méthane en donnant la sélectivité en hydrocarbures $C_{5+}$ parmi les hydrocarbures supérieurs recueillis.

Tableau II

| Adsorption | | | | | Hydrogénation | | | | Sélectivité |
|---|---|---|---|---|---|---|---|---|---|
| Température (°C) | Pression (Pa) | Débit (ml/min) | Durée (secondes) | $CH_4$ passé sur catalyseur (mg) | Température (°C) | Pression (Pa) | Débit (ml/min) | Durée (minutes) | $(C_{5+}/C_{2+}$, en %) |
| 300 | $4.10^5$ | 400 | 36 | 171 | 300 | $16.10^5$ | 200 | 10 | 82,9 |
| 300 | $4.10^5$ | 2400 | 60 | 1710 | 300 | $4.10^5$ | 200 | 10 | 67,8 |
| 300 | $16.10^5$ | 1600 | 36 | 686 | 300 | $16.10^5$ | 200 | 10 | 87,5 |

Ce tableau montre bien qu'en réalisant l'étape d'adsorption à des pressions élevées et l'étape de désorption à température constante, on obtient une bonne conversion du méthane en hydrocarbures lourds liquides de type $C_{5+}$.

Ce tableau illustre également l'effet positif d'une pression élevée lors de l'étape de désorption: la sélectivité de la conversion du méthane est nettement meilleure lorsque cette étape est réalisée à $16.10^5$ Pa que lorsqu'elle est réalisée à $4.10^5$ Pa.

EXEMPLE 3

Cet exemple illustre la possibilité d'utiliser différents types de catalyseurs, dans différentes conditions, avec désorption à température constante (Tableau III) ou désorption polytherme (Tableau IV). Dans les deux cas, la désorption est réalisée par hydrogénation, sous pression.

Les tableaux III et IV ci-après rassemblent, pour différentes conditions opératoires, les résultats de la conversion du méthane en donnant la sélectivité des hydrocarbures $C_{5+}$ recueillis.

Les supports catalytiques testés ici sont le support NaY, qui est une zéolithe de type "faujasite" dont la surface spécifique est de 600 $m^2$/g, et le support $SiO_2$ qui est une silice dont la surface spécifique est de 1000 $m^2$/g. Le débit gazeux est un débit volumique mesuré aux conditions normales de température et de pression.

Ces tableaux montrent que certains catalyseurs conduisent à une meilleure sélectivité que d'autres : en particulier les catalyseurs sur zéolithe quelle que soit leur teneur en métaux ou les catalyseurs sur silice possédant une teneur suffisante en métaux.

Tableau III

| Catalyseur | Adsorption | | | | | Hydrogénation | | | | Sélectivité |
|---|---|---|---|---|---|---|---|---|---|---|
| | Température (°C) | Pression (Pa) | Débit (ml/min) | Durée (secondes) | $CH_4$ envoyé sur catalyseur (mg) | Température (°C) | Pression (Pa) | Débit (ml/min) | Durée (minutes) | $(C_{5+}/C_2$, en %) |
| 15 % Ni/NaY | 300 | $5.10^5$ | 400 | 60 | 286 | 300 | $5.10^5$ | 200 | 10 | 33,3 |
| 15 % Co/NaY | 300 | $5.10^5$ | 400 | 60 | 286 | 300 | $5.10^5$ | 200 | 10 | 34,1 |
| 5 % Rh/NaY | 250 | $5.10^5$ | 400 | 300 | 1429 | 250 | $5.10^5$ | 200 | 10 | 65,9 |
| 6 % Pt/NaY | 320 | $5.10^5$ | 400 | 60 | 286 | 320 | $5.10^5$ | 50 | 10 | 70 |
| 6 % Pt/SiO$_2$ | 320 | $5.10^5$ | 400 | 60 | 286 | 320 | $5.10^5$ | 200 | 10 | 31 |
| 15 % Ni/SiO$_2$ | 300 | $5.10^5$ | 400 | 60 | 286 | 300 | $5.10^5$ | 200 | 10 | 87 |

EP 0 858 987 B1

Tableau IV

| Catalyseur | Adsorption | | | | | Hydrogénation polytherme | | Sélectivité |
|---|---|---|---|---|---|---|---|---|
| | Température (°C) | Pression (Pa) | Débit (ml/min) | Durée (secondes) | $CH_4$ envoyé sur catalyseur (mg) | Pression (Pa) | Débit (ml/min) | ($C_{5+}/C_{2+}$ en %) |
| 15 % Ni/NaY | 300 | $5.10^5$ | 400 | 60 | 286 | $5.10^5$ | 200 | 63 |
| 15 % Ni/NaY | 400 | $5.10^5$ | 400 | 60 | 286 | $5.10^5$ | 200 | 45 |
| 5 % Rh/NaY | 250 | $5.10^5$ | 400 | 300 | 1429 | $5.10^5$ | 200 | 71,1 |
| 5 % Rh/NaY | 300 | $5.10^5$ | 400 | 60 | 1429 | $5.10^5$ | 200 | 58,5 |
| 15 % Co/NaY | 250 | $5.10^5$ | 400 | 60 | 286 | $5.10^5$ | 200 | 42 |
| 15 % Co/NaY | 400 | $5.10^5$ | 400 | 60 | 286 | $5.10^5$ | 200 | 59,6 |

EXEMPLE 4

Cet exemple illustre l'intérêt de réaliser avantageusement l'étape de désorption hydrogénante à une température inférieure à celle de l'étape d'adsorption. Cette température est constante durant l'étape d'hydrogénation.

Le tableaux V ci-après rassemble, pour différentes conditions opératoires et en utilisant 100 mg du catalyseur de l'exemple 1, les résultats de la conversion du méthane en donnant la sélectivité des hydrocarbures $C_{5+}$ recueillis. Le débit gazeux est un débit volumique mesuré aux conditions normales de température et de pression.

Tableau V

| Adsorption | | | | | Hydrogénation | | | | Sélectivité |
|---|---|---|---|---|---|---|---|---|---|
| Température (°C) | Pression (Pa) | Débit (ml/min) | Durée (secondes) | $CH_4$ envoyé sur catalyseur (mg) | Température (°C) | Pression (Pa) | Débit (ml/min) | Durée (minutes) | ($C_{5+}$ /$C_{2+}$ en %) |
| 350 | $16.10^5$ | 1600 | 6 | 114 | 350 | $16.10^5$ | 200 | 10 | 33 |
| 350 | $16.10^5$ | 1600 | 6 | 114 | 325 | $16.10^5$ | 200 | 10 | 53 |
| 350 | $16.10^5$ | 1600 | 15 | 286 | 350 | $16.10^5$ | 200 | 10 | 43 |
| 350 | $16.10^5$ | 1600 | 15 | 286 | 325 | $16.10^5$ | 200 | 10 | 55 |
| 400 | $16.10^5$ | 1600 | 3 | 57 | 400 | $16.10^5$ | 200 | 10 | 5 |
| 400 | $16.10^5$ | 1600 | 3 | 57 | 300 | $16.10^5$ | 200 | 10 | 51,7 |

Ce tableau montre bien que lorsque l'étape d'hydrogénation est réalisée à une température inférieure à celle de l'étape d'adsorption, la conversion du méthane en hydrocarbures lourds liquides de type $C_{5+}$ est meilleure.

**Revendications**

1. Procédé de conversion d'un mélange d'hydrocarbures légers, gazeux aux conditions normales de température et de pression, contenant du méthane, en hydrocarbures supérieurs, liquides aux conditions normales de température et de pression, ce procédé comportant deux étapes, une étape d'adsorption dudit mélange sur un catalyseur supporté métallique, suivie d'une étape de désorption des espèces adsorbées, et étant caractérisé en ce que l'étape d'adsorption est réalisée à température supérieure ou égale à 300 °C et en ce que au moins l'une des deux étapes est réalisée sous une pression supérieure ou égale à $5.10^5$ Pa.

2. Procédé selon la revendication 1, caractérisé en ce que les deux étapes sont réalisées sous pression supérieure ou égale à $5.10^5$ Pa.

3. Procédé selon la revendication 1, caractérisé en ce que seule ladite étape d'adsorption est réalisée sous pression supérieure ou égale à $5.10^5$ Pa.

4. Procédé selon la revendication 1, caractérisé en ce que seule ladite étape de désorption est réalisée sous pression supérieure ou égale à $5.10^5$ Pa.

5. Procédé selon l'une quelconque des revendications précédentes, caractérisé en ce que les étapes effectuées sous pression supérieure ou égale à $5.10^5$ Pa sont réalisées sous une pression comprise entre $10.10^5$ Pa et $80.10^5$ Pa.

6. Procédé selon l'une quelconque des revendications précédentes, caractérisé en ce que l'étape d'adsorption est réalisée à température comprise entre 350 et 500°C.

7. Procédé selon l'une quelconque des revendications précédentes, caractérisé en ce que l'étape de désorption s'effectue à température supérieure ou égale à 100 °C.

8. Procédé selon la revendication 7, caractérisé en ce que l'étape de désorption s'effectue à une température constante, comprise entre 100 et 400°C.

9. Procédé selon la revendication 7, caractérisé en ce que l'étape de désorption s'effectue en augmentant progressivement la température dans l'intervalle de température compris entre 100 et 600 °C.

10. Procédé selon l'une quelconque des revendications précédentes, caractérisé en ce que l'étape d'adsorption s'effectue pendant une durée comprise entre 3 secondes et 2 minutes.

11. Procédé selon l'une quelconque des revendications précédentes, caractérisé en ce que l'étape de désorption s'effectue pendant une durée comprise entre 1 seconde et 10 minutes, et de préférence comprise entre 5 secondes et 2 minutes.

12. Procédé selon l'une quelconque des revendications précédentes, caractérisé en ce que le catalyseur supporté métallique comprend un métal ou un alliage de métaux dont l'un au moins est choisi dans le groupe VIII de la Classification Périodique des éléments.

13. Procédé selon l'une quelconque des revendications précédentes, caractérisé en ce que le catalyseur supporté métallique comprend un métal choisi dans le groupe constitué par le platine, le cobalt, le nickel, le rhodium, le cuivre et les alliages de ces métaux.

14. Procédé selon la revendication 13, caractérisé en ce que le catalyseur est un catalyseur supporté nickel/cuivre.

15. Procédé selon l'une quelconque des revendications 12 à 14, caractérisé en ce que le catalyseur présente une teneur en métal comprise entre 5 et 50 % en poids par rapport au poids du support.

16. Procédé selon l'une quelconque des revendications 12 à 15, caractérisé en ce que le catalyseur présente une surface spécifique supérieure ou égale à 200 $m^2$/g et/ou un volume poreux supérieur ou égal à 0,2 $cm^3$/g.

17. Procédé selon l'une quelconque des revendications 12 à 16, caractérisé en ce que le support du catalyseur est choisi dans le groupe constitué par les oxydes de gallium, de titane, de thorium, de bore, les mélanges de ces oxydes, les silices, les alumines, les silice-alumines, les silicalites, les alumino-silicates, les magnésies et les zircones.

**18.** Procédé selon l'une quelconque des revendications précédentes, caractérisé en ce que l'hydrogène formé durant l'étape d'adsorption est, partiellement ou pour l'essentiel, éliminé du milieu réactionnel in situ par un dispositif connu en soi, tel qu'un dispositif membranaire.

**19.** Procédé selon l'une quelconque des revendications précédentes, caractérisé en ce que la désorption est une désorption hydrogénante.

**20.** Procédé selon la revendication 19, caractérisé en ce que la désorption hydrogénante s'effectue au moyen d'hydrogène pur, ou en mélange avec un ou plusieurs hydrocarbure(s) et/ou un ou plusieurs gaz inerte(s).

**21.** Procédé selon l'une quelconque des revendications précédentes, caractérisé en ce que les hydrocarbures produits et se trouvant l'état non liquide aux conditions normales de température et de pression, sont recyclés partiellement ou totalement, en mélange avec le mélange d'hydrocarbures légers constituant la charge, ou séparément.

**Patentansprüche**

**1.** Verfahren zur Umsetzung eines Gemisches aus leichten Kohlenwasserstoffen, die unter normalen Temperatur- und Druckbedingungen gasförmig sind und Methan enthalten, in höheren Kohlenwasserstoffen, die unter normalen Temperatur- und Druckbedingungen flüssig sind, welches zwei Arbeitsschritte umfaßt: einen Schritt zur Adsorption des Gemisches auf einem metallischen Katalysator mit Träger und einen anschließenden Schritt zur Desorption der adsorbierten Arten, **dadurch gekennzeichnet**, daß der Schritt zur Adsorption bei einer Temperatur von mindestens 300 °C vorgenommen wird, und daß mindestens einer der beiden Schritte unter einem Druck von mindestens $5 \cdot 10^5$ Pa ausgeführt wird.

**2.** Verfahren nach Anspruch 1, dadurch gekennzeichnet, daß die beiden Schritte unter einem Druck von mindestens $5 \cdot 10^5$ Pa ausgeführt werden.

**3.** Verfahren nach Anspruch 1, dadurch gekennzeichnet, daß nur der Schritt zur Adsorption unter einem Druck von mindestens $5 \cdot 10^5$ Pa ausgeführt wird.

**4.** Verfahren nach Anspruch 1, dadurch gekennzeichnet, daß nur der Schritt zur Desorption unter einem Druck von mindestens $5 \cdot 10^5$ Pa ausgeführt wird.

**5.** Verfahren nach einem der vorhergehenden Ansprüche, dadurch gekennzeichnet, daß die unter einem Druck von mindestens $5 \cdot 10^5$ Pa ausgeführten Schritte unter einem Druck im Bereich zwischen $10 \cdot 10^5$ Pa und $80 \cdot 10^5$ Pa ausgeführt werden.

**6.** Verfahren nach einem der vorhergehenden Ansprüche, dadurch gekennzeichnet, daß der Schritt zur Adsorption bei einer Temperatur im Bereich Zwischen 350 und 500 °C ausgeführt wird.

**7.** Verfahren nach einem der vorhergehenden Ansprüche, dadurch gekennzeichnet, daß der Schritt zur Desorption bei einer Temperatur von mindestens 100 °C ausgeführt wird.

**8.** Verfahren nach Anspruch 7, dadurch gekennzeichnet, daß der Schritt zur Desorption bei einer konstanten Temperatur im Bereich zwischen 100 und 400 °C ausgeführt wird.

**9.** Verfahren nach Anspruch 7, dadurch gekennzeichnet, daß der Schritt zur Desorption unter allmählicher Erhöhung der Temperatur im Temperaturbereich zwischen 100 und 600 °C ausgeführt wird.

**10.** Verfahren nach einem der vorhergehenden Ansprüche, dadurch gekennzeichnet, daß der Schritt zur Adsorption während eines Zeitraums von 3 Sekunden bis 2 Minuten ausgeführt wird.

**11.** Verfahren nach einem der vorhergehenden Ansprüche, dadurch gekennzeichnet, daß der Schritt zur Desorption während eines Zeitraums von 1 Sekunde bis 10 Minuten und vorzugsweise von 5 Sekunden bis 2 Minuten ausgeführt wird.

12. Verfahren nach einem der vorhergehenden Ansprüche, dadurch gekennzeichnet, daß der metallische Katalysator mit Träger ein Metall oder eine Legierung aus Metallen aufweist, von denen mindestens eines aus der Gruppe VIII des Periodensystems gewählt ist.

13. Verfahren nach einem der vorhergehenden Ansprüche, dadurch gekennzeichnet, daß der metallische Katalysator mit Träger ein Metall umfaßt, das aus der von Platin, Kobalt, Nickel, Rhodium, Kupfer und Legierungen dieser Metalle gebildeten Gruppe gewählt ist.

14. Verfahren nach Anspruch 13, dadurch gekennzeichnet, daß der Katalysator ein Nickel-/Kupfer-Katalysator mit Träger ist.

15. Verfahren nach einem der Ansprüche 12 bis 14, dadurch gekennzeichnet, daß der Katalysator einen Metallgehalt zwischen 5 und 50 Gew. %, bezogen auf das Gewicht des Trägers, aufweist.

16. Verfahren nach einem der Ansprüche 12 bis 15, dadurch gekennzeichnet, daß der Katalysator eine spezifische Oberfläche von mindestens 200 $m^2$/g und/oder ein Porenvolumen von mindestens 0,2 $cm^3$/g aufweist.

17. Verfahren nach einem der Ansprüche 12 bis 16, dadurch gekennzeichnet, daß der Träger des Katalysators aus der Gruppe gewählt ist, die aus Gallium-, Titan-, Thorium-, Bor- Oxiden, Gemischen dieser Oxide, Siliziumoxiden, Aluminiumoxiden, Siliziumoxid-Aluminiumoxiden, Silikaliten, Aluminosilikaten, Magnesiumoxiden und Zirkonen gebildet wird.

18. Verfahren nach einem der vorhergehenden Ansprüche, dadurch gekennzeichnet, daß der während des Adsorptionsschritts gebildete Wasserstoff teilweise oder im wesentlichen aus dem Reaktionsmedium in situ mittels einer an sich bekannten Vorrichtung wie beispielsweise einer Vorrichtung mit Membran entfernt wird.

19. Verfahren nach einem der vorhergehenden Ansprüche, dadurch gekennzeichnet, daß es sich bei der Desorption um eine Desorption mit Umsetzung in Wasserstoff handelt.

20. Verfahren nach Anspruch 19, dadurch gekennzeichnet, daß die Desorption mit Umsetzung in Wasserstoff mit Hilfe von reinem Wasserstoff oder mit einem Gemisch mit einem oder mehreren Kohlenwasserstoffen und/oder einem oder mehreren Edelgasen durchgeführt wird.

21. Verfahren nach einem der vorhergehenden Ansprüche, dadurch gekennzeichnet, daß die gebildeten Kohlenwasserstoffe, die unter normalen Temperatur- und Druckbedingungen im nicht-flüssigen Zustand vorliegen, teilweise oder ganz in einem Gemisch mit dem den Einsatz bildenden Gemisch aus leichten Kohlenwasserstoffen oder separat rückgeführt werden.


**Claims**

1. Process for the conversion of a mixture of light hydrocarbons, gaseous under normal temperature and pressure conditions and containing methane, into higher hydrocarbons, which are liquid under normal temperature and pressure conditions, this process comprising two stages, a stage comprising adsorption of said mixture on a supported metallic catalyst followed by a stage comprising desorption of the adsorbed species, and being characterised in that the adsorption stage is performed at a temperature higher than or equal to 300 °C and in that at least one of the two stages is performed under a pressure greater than or equal to $5.10^5$ Pa.

2. Process according to claim 1, characterised in that both stages are performed under a pressure greater than or equal to $5.10^5$ Pa.

3. Process according to claim 1, characterised in that only said adsorption stage is performed under a pressure greater than or equal to $5.10^5$ Pa.

4. Process according to claim 1, characterised in that only said desorption stage is performed under a pressure greater than or equal to $5.10^5$ Pa.

5. Process according to any one of the preceding claims, characterised in that the stages carried out under a pressure greater than or equal to $5.10^5$ Pa are performed under a pressure of between $10.10^5$ Pa and $80.10^5$ Pa.

6. Process according to any one of the preceding claims, characterised in that the adsorption stage is performed at a temperature of between 350 and 500 °C.

7. Process according to any one of the preceding claims, characterised in that the desorption stage is carried out at a temperature higher than or equal to 100 °C.

8. Process according to claim 7, characterised in that the desorption stage is carried out at a constant temperature, of between 100 and 400 °C.

9. Process according to claim 7, characterised in that the desorption stage is carried out with a progressive increase in temperature within the temperature range of between 100 and 600 °C.

10. Process according to any one of the preceding claims, characterised in that the adsorption stage is carried out for a period of between 3 seconds and 2 minutes.

11. Process according to any one of the preceding claims, characterised in that the desorption stage is carried out for a period of between 1 second and 10 minutes, and preferably between 5 seconds and 2 minutes.

12. Process according to any one of the preceding claims, characterised in that the supported metallic catalyst comprises a metal or an alloy of metals, at least one of which is selected from group VIII of the Periodic System of Elements.

13. Process according to any one of the preceding claims, characterised in that the supported metallic catalyst comprises a metal selected from the group consisting of platinum, cobalt, nickel, rhodium, copper and alloys of these metals.

14. Process according to claim 13, characterised in that the catalyst is a supported nickel/copper catalyst.

15. Process according to any one of claims 12 to 14, characterised in that the catalyst has a metal content of between 5 and 50 wt.% relative to the weight of the support.

16. Process according to any one of claims 12 to 15, characterised in that the catalyst has a specific surface area greater than or equal to 200 $m^2$/g and/or a porous volume greater than or equal to 0.2 $cm^3$/g.

17. Process according to any one of claims 12 to 16, characterised in that the catalyst support is selected from the group consisting of the oxides of gallium, titanium, thorium, boron, mixtures of these oxides, silicas, aluminas, silica/aluminas, silicalites, alumino-silicates, magnesias and zirconias.

18. Process according to any one of the preceding claims, characterised in that the hydrogen formed during the adsorption stage is, in part or in the main, eliminated from the reaction medium in situ by a device known per se, such as a membrane device.

19. Process according to any one of the preceding claims, characterised in that the desorption stage comprises hydrogenating desorption.

20. Process according to claim 19, characterised in that the hydrogenating desorption is performed using pure hydrogen or hydrogen mixed with one or more hydrocarbon(s) and/or one or more inert gas(es).

21. Process according to any one of the preceding claims, characterised in that the hydrocarbons produced and in a non-liquid state under normal temperature and pressure conditions are recycled in part or in total, either mixed with the mixture of light hydrocarbons constituting the charge or separately.